# EUROPÄISCHE PATENTSCHRIFT

(11) **EP 1 029 241 B1**
(45) Veröffentlichungstag und Bekanntmachung des Hinweises auf die Patenterteilung: **01.09.2004**
(21) Anmeldenummer: 98962216.2
(22) Anmeldetag: 06.11.1998
(51) Int. Cl.: G01N 33/50, C12Q 1/02, C12Q 1/42, C12Q 1/54

(54) **VERFAHREN ZUR PARALLELEN BESTIMMUNG DER ZELLVITALITÄT UND GENTRANSFER-EFFIZIENZ**
PROCESS FOR DETERMINING CELL VITALITY AND EFFICIENCY IN PARALLEL AFTER A GENE TRANSFER INTO EUKARYOTIC CELLS AND DUAL TEST SYSTEM
PROCEDE SERVANT A DETERMINER LA VITALITE DE CELLULES ET PARALLELEMENT L'EFFICACITE APRES UN TRANSFERT DE GENES DANS DES CELLULES EUCARYOTES ET DOUBLE SYSTEME DE DETERMINATION

(30) Priorität: 07.11.1997 DE 19750790; 13.02.1998 DE 19805818
(43) Veröffentlichungstag der Anmeldung: 23.08.2000
(73) Patentinhaber: MAX-DELBRÜCK-CENTRUM FÜR MOLEKULARE MEDIZIN, 13125 Berlin (DE)
(72) Erfinder: GROTH, Detlef, D-14548 Ferch (DE); RESZKA, Regina, D-16341 Schwanebeck (DE)
(74) Vertreter: Baumbach, Friedrich, Dr.
(86) Internationale Anmeldenummer: PCT/DE1998/003245
(87) Internationale Veröffentlichungsnummer: WO 1999/024602

(56) Entgegenhaltungen:
- CHEMICAL ABSTRACTS, vol. 129, no. 1, 6. Juli 1998 Columbus, Ohio, US; abstract no. 2317, XP002101804 & D. GROTH ET AL.: "Transfection assay for dual determination of toxicity and gene expression." ANALYTICAL BIOCHEMISTRY, Bd. 258, Nr. 1, 10. April 1998, Seiten 141-143, New York NY USA
- CHEMICAL ABSTRACTS, vol. 111, no. 13, 25. September 1989 Columbus, Ohio, US; abstract no. 110297, XP002101805 in der Anmeldung erwähnt & K. LIM ET AL.: "A simple assay for DNA transfection by incubation of the cells in culture dishes with substrates for beta-galactosidase." BIOTECHNIQUES, Bd. 76, 1989, Seiten 576-579, Chapel Hill NC USA
- CHEMICAL ABSTRACTS, vol. 104, no. 7, 17. Februar 1986 Columbus, Ohio, US; abstract no. 48073, XP002101806 in der Anmeldung erwähnt & D.T. CONNOLLY ET AL.: "Determination of the number of endothelial cells in culture using an acid phosphatase assay." ANALYTICAL BIOCHEMISTRY, Bd. 152, Nr. 1, 1986, Seiten 136-140, New York NY USA

## Beschreibung

Die Erfindung betrifft ein Verfahren zur parallelen Bestimmung von Zellvitalität und Effizienz unter Verwendung eines Reportergens nach Gentransfer in eukaryontische Zellen im selben Kulturgefäß sowie ein duales Testsystem.

Die Applikation von DNA in eukaryontische Zellen ist eine bedeutende Technik der Molekularbiologie. Für diesen Zweck wurden eine Reihe von verfahren, wie die Kalziumphosphatpräzipationsmethode (Graham-FL; Eb-AJ-van-der (1973). A new technique for the assay of infectivity of human adenovirus 5 DNA. Virology 52: 456-67), verschiedene virusvermittelte Methoden (Rosen-CA; Sodroski-JG; Haseltine-WA ( 1985). The location of cis-acting regulatory sequences in the human T cell lymphotropic virus type III (HTLV-III/LAV) long terminal repeat. Cell 41: 813-23), die Applikation von DNA mit Hilfe kationischer Liposomen (Felgner-PL; Gadek-TR; Holm-M; Roman-R; Chan-HW; Wenz-M; Northrop-JP; Ringold-GM; Danielsen-M (1987). Lipofection: a highly efficient, lipid-mediated DNA-transfection procedure. Proc-Natl-Acad-Sci-U-S-A. 84: 7413-7) bzw. von Polymeren (Fauci-AS (1986). Current issues in developing a strategy for dealing with the acquired immunodeficiency syndrome. Proc-Natl-Acad-Sci-U-S-A. 83: 9278-83; Mosca-JD; Bednarik-DP; Raj-NB; Rosen-CA; Sodroski-JG; Haseltine-WA; Pitha-PM (1987). Herpes simplex virus type-1 can reactivate transcription of latent human immunodeficiency virus. Nature. 325: 67-70; Gendelman-HE: Phelps-W; Feigenbaum-L; Ostrove-JM; Adachi-A; Howley-PM; Khoury-G; Ginsberg-HS; Martin-MA (1986). Transactivation of the human immunodeficiency virus long terminal repeat sequence by DNA viruses. Proc-Natl-Acad-Sci-U-S-A. 83: 9759-63) und mit Hilfe physikalischer Methoden (O'Hare-P; Hayward-GS (1985). Evidence for a direct role for both the 175,000- and 110,000-molecular-weight immediate-early proteins of herpes simplex virus in the transactivation of delayed-early promoters. J-Virol. 53: 751-60, Gorman-CM; Moffat-LF; Howard-BH (1982). Recombinant genomes which express chloramphenicol acetyltransferase in mammalian cells. Mol-Cell-Biol. 2: 1044-51) beschrieben.
Um die Effizienz der Genapplikation bei in vitro Anwendungen zu untersuchen, werden eine Reihe sogenannter Reportergene wie das LacZ-Gen (Lim-K; Chae-CB (1989). A simple assay for DNA transfection by incubation of the cells in culture dishes with substrates for beta-galactosidase. Biotechniques. 7: 576-9), das Luziferase-Gen (Nordeen-SK (1988). Luciferase reporter gene vectors for analysis of promoters and enhancers. Biotechniques. 6: 454-8), das Chloramphenicolacetyltransferase-Gen (Gorman et al (1982), s.o.) und andere verwandt. Das LacZ-Gen bietet gegenüber anderen Reportergenen verschiedene Vorteile. Zum ersten ist es möglich, einzelne, das Reportergen exprimierende Zellen, die transfizierten Zellen, mit Hilfe der sogenannten X-Gal-Färbung (Lojda-Z; Slaby-J; Kraml-J; Kolinska-J (1973). Synthetic substrates in the histochemical demonstration of intestinal disaccharidases. Histochemie. 34: 361-9) anzufärben. Desweiteren ist die Bestimmung der Gesamtgenexpression einer größeren Anzahl von Zellen mit Hilfe eines einfachen Farbtestes (Lim und Chae, 1989, s.o.) bestimmbar. Diese Bestimmungen der Genexpression sind im Gegensatz zu einer Vielzahl anderer Reportergene in jedem Laboratorium durchführbar, da keinerlei radioaktive oder luminometrische Messungen sondern lediglich die Messung der Farbentwicklung notwendig ist.
Da die meisten Techniken, die DNA in eukaryontische Zellen applizieren, von einer gewissen Zellschädigung begleitet sind, ist die Bestimmung der Zellvitalität nach Gentransfer, bzw. die Bestimmung der Toxizität, von Bedeutung für die Charakterisierung der Effizienz und Sicherheit der Methode. Für die Bestimmung der Toxizität von Verbindungen existieren eine Reihe von Verfahren, die zumeist die Messung der Aktivität eines repräsentativen Enzyms der Zellen oder die manuelle Auszählung der lebenden Zellen zum Inhalt haben. Für die Untersuchung einer großen Probenzahl haben sich die enzymatischen Methoden weitestgehend durchgesetzt. Von Bedeutung ist hierbei insbesondere der sogenannte MTT-Test (Mosmann-T (1983). Rapid colorimetric assay for cellular growth and survival: application to proliferation and cytotoxicity assays. J-Immunol-Methods. 65: 55-63) sowie der Saure Phosphatasetest (Connolly-DT; Knight-MB; Harakas-NK; Wittwer-AJ; Feder-J (1986). Determination of the number of endothelial cells in culture using an acid phosphatase assay. Anal-Biochem. 152: 136-40), bei denen die Aktivität mitochondrialer Dehydrogenasen (MTT) bzw. zytosolischer saurer Phosphatasen bestimmt wird. Bis jetzt existieren nur Verfahren, die in separaten Ansätzen entweder die Effizienz der Methode über die Reportergenaktivität bestimmen oder Methoden, die die Toxizität der Methode über eine für die Zellzahl repräsentative Enzymaktivität feststellen.

Gemäß der Erfindung ist es überraschend möglich, für die gleichzeitige Bestimmung der Zellvitalität und der Expression des Reportergenes einen modifizierten Saure Phosphatase Assay mit einem colorimetrischen oder fluorimetrischen Reportergen Assay, vorzugsweise Beta-Galaktosidase-Assay, zu kombinieren.

Die Erfindung wird gemäß den Ansprüchen realisiert.
Dazu werden die Zellen nach Expression des Reportergens (24-72h nach der Transfektion) mit Phosphat gepufferter Salzlösung (PBS) gewaschen. Anschließend erfolgt die Zugabe des Puffers zur Bestimmung der Sauren Phosphatase, welcher vorzugsweise p-Nitrophenylphosphat (pNPP) als Substrat, 2-Morpholinoethanol-sulfonsäure (MES) als Puffer und Alkylphenylpolyethylenglycol (Triton X-100) oder Naphtylphosphat in Natriumacetat/Triton X-100 zur Zellyse enthält. Nach 15 bis 30 min Inkubation bei 37 °C wird die Reaktion durch Zugabe von Tris (hydroxymethyl)aminomethane-HCl (pH 8.0) (Tris-HCl) oder HEPES abgestoppt und die Menge entweder an dem Reaktionsprodukt (p-Nitrophenol)-spektrophotometrisch bei 405 nm oder fluorimetrisch bestimmt. Die Höhe der bestimmten Saure Phosphatase-Aktivität ist ein Maß für die Anzahl lebender Zellen.

Anschließend erfolgt die Zugabe eines Beta-Galaktosidase-Assay Puffers in die gleiche Zellkulturschale. Dieser Puffer enthält CPRG bzw. ONPG in Hank's Balancierter Salzlösung (HBSS) oder/und MUG in HBSS, Auszug aus MUG-Stammlösung in DMSO und HBSS. Nach einer Inkubationszeit von 2 min bis zu 24 h wird die Menge an den Reaktionsprodukten spektrophotometrisch bei 540-580 nm oder fluorimetrisch bestimmt. So ist z.B. die Menge an Chlorphenolrot ein Maß für die Expression des Reportergens.

Ein duales Testsystem zur parallelen Bestimmung von Zellvitalität und Effizienz nach Gentransfer in eukaryontische Zellen umfaßt
a) einen Vitalitäts-Assay mit
   einem Substrat zur Bestimmung Saurer Phosphatase und einem Puffer für die Zellyse, wobei als Substrat pNPP oder Naphthylphosphat verwendet wird und der verwendete Puffer eine niedrige Ionenstärke besitzt, die nach Bestimmung der Saure Phosphataseaktivität eine Veränderung des pH-Wertes vom sauren in den basischen pH-Bereich ermöglicht, und
b) einem Substrat zur colorimetrischen oder.fluorimetrischen Bestimmung der β-Galaktosidase-Aktivität als Reportengen-Assay zur Effizienzbestimmung.

Der erfindungsgemäße Test erlaubt zum ersten Mal die Bestimmung von Effizienz und Toxizität einer Gentransfermethode in einem, Ansatz und auch direkt im Zellkulturgefäß. Das dabei genutzte Verfahren verwendet die Aktivität des Enzyms Saure Phosphatase als Marker für die Vitalität der Zellen nach Gentransfer und die Aktivität des Genprodukts des applizierten lacZ-Gens, die Beta-Galaktosidaseaktivität, als Marker für die Effizienz der Genapplikation. Im ersten Schritt erfolgt die Bestimmung der Saure Phosphataseaktivität mit Hilfe eines modifizierten

Assays. Das der Sauren Phosphatase bevorzugt angebotene Substrat pNPP wird dabei in p-Nitrophenol umgewandelt und die Menge an Reaktionsprodukt photometrisch durch die Lichtabsorption bei 405 nm bestimmt. Der bei dieser Bestimmung verwendete Puffer enthält eine im Vergleich zu bisherigen Methoden geringere Konzentration an Puffersubstanz und ist geeignet für die Bestimmung der Zellaktivität. Im weiteren erlaubt die niedrige Ionenstärke des verwendeten Puffers, nach Bestimmung der Saure Phosphataseaktivität, eine Veränderung des pH-Wertes von 5,5 zu ca. 7,8 durch Zugabe eines Überschusses an Tris-HCl Puffer oder HEPES-Puffer (pH 8,0). Dieses Puffergemisch ist, nach Zugabe eines Beta-Galaktosidasetestpuffers, für die Bestimmung der im Ansatz befindlichen Beta-Galaktosidaseaktivität geeignet. Als Substrate werden CPRG, ONPG oder MUG verwendet. Das Absorptionsmaximum des Reaktionsproduktes der Beta-Galaktosidasereaktion bei Verwendung von CPRG, Chlorphenolrot, liegt bei etwa 570 nm. Damit stört das Spektrum des bereits im Ansatz befindlichen Reaktionsproduktes der Saure Phosphatasereaktion p-Nitrophenol nicht die folgende Bestimmung. Das Hydrolyseprodukt o-Nitrophenol hat im Vergleich zu p-Nitrophenol, dem Reaktionsprodukt der Saure Phosphatasereaktion, ein in den längerwelligen Bereich des Lichts verschobenes Absorptionspektrum. Dies ermöglicht die Bestimmung der Beta-Galaktosidasereaktion z. B. bei 490nm. Methylumbelliferon als Reaktionsprodukt der Beta-Galaktosidasereaktion bei Verwendung von MUG, kann fluorimetrisch bestimmt werden. Auch die Kombination zweier fluoreszenzaktiver Substrate ist möglich, wenn sich die Emissionspektren nicht überlagern. So können Naphtylphosphat als Substrat der Saure Phosphatasereaktion und MUG als Substrat der Beta-Galaktosidasereaktion verwendet werden.
Die Hemmung der Beta-Galaktosidasebestimmung durch die vorhergehende Aktivitätsmessung des Enzyms Saure Phosphatase beträgt weniger als eine Zehnerpotenz und ist akzeptabel, da das Beta-Galaktosidaseenzym bei den verwendeten Bedingungen recht stabil ist und die Sensitivität des Testes durch verlängerte Inkubationszeiten weiter gesteigert werden kann. Das erfindungsgemäße Verfahren kann für die Charakterisierung von Gentransfermethoden hinsichtlich Toxizität und Effizienz verwendet werden. Der beschriebene Saure Phosphatasetest ist bei der Zellzahlbestimmung schneller und genauer als der weithin genutzte MTT-Test. Desweiteren ist der Gebrauch toxischer Chemikalien reduziert. Im Gegensatz zu bisher beschriebenen Methoden werden in dem Verfahren hier Puffer mit niedriger Ionenstärke verwendet. Dies beeinflußt nicht die Sensitivität der Methode, erlaubt aber eine anschließende Erhöhung des pH-Wertes durch Zugabe eines höherionischen schwach alkalischen Puffersystems. Die dabei erhaltenen Bedingungen sind günstig für die nachfolgende Bestimmung des zweiten Enzyms im Reaktionsansatz, des Beta-Galaktosidaseenzyms. Die Aktivität dieses Enzyms ist durch die vorausgehende Bestimmung der Sauren Phosphatase nur geringfügig vermindert und für die Bestimmung der Reportergenexpression in sämtlichen getesteten Zellinien und in primären Cornea Endothel Zellen des Rindes hoch genug. Dieser Test ist sehr geeignet für ein umfassendes schnelles und preiswertes Testen der verschiedenen Methoden bzw. Stoffe für eine Genapplikation.

Durch Ausführungsbeispiele wird die Erfindung näher erläutert.

### Abkürzungen:

- MUG:: Methylumbelliferyl-β-D-Galaktopyranosid
- ONPG:: o-Nitrophenol-β-D-Galaktopyranosid
- PBS:: Phosphat gepufferte Saline
- CPRG:: Chlorphenolrot-β-D-Galaktopyranosid
- pNPP:: p-Nitrophenylphosphat
- pNP:: p-Nitrophenol
- DMSO:: Dimethylsulfoxid
- HBSS:: Hank's Balancierte Salzlösung

### Methoden:

### Bestimmung der Absorptionspektren von p-Nitrophenol, o-Nitrophenol und Chlorphenolrot.

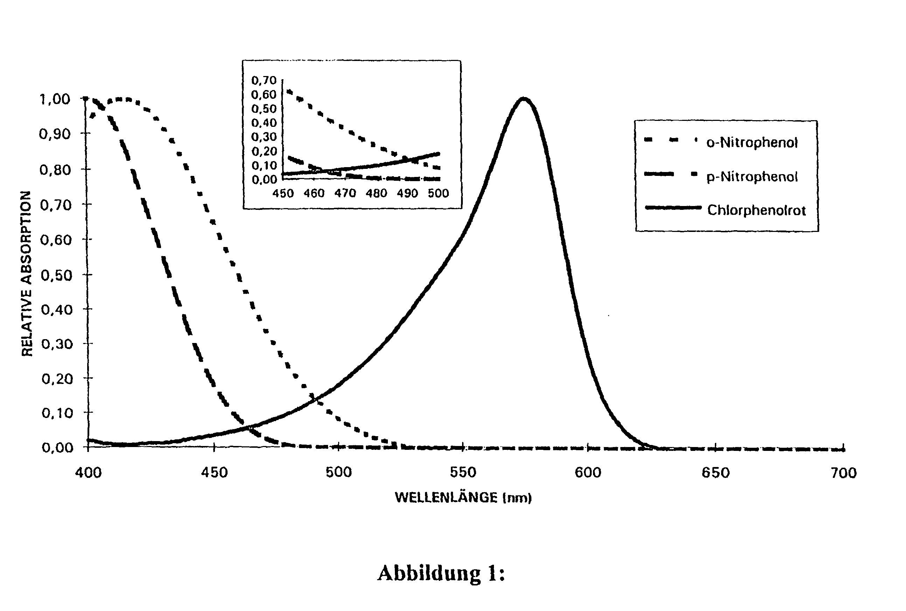

Das Substrat pNPP wird in der Zellkultur in p-Nitrophenol umgewandelt. Dazu wird zu einer MCF-7 Zellkultur die Substratlösung 10 mM pNPP in 0.02 M MES (pH 5.5) und 0.1 % Triton X-100 für die Zellyse gegeben. Nach einstündiger Inkubation wird der Überstand abgenommen und die Zelltrümmer werden durch Zentrifugation entfernt. Danach erfolgt die Aufnahme des Spektrums an einem Spektrophotometer.
Die Substrate ONPG und CPRG werden durch die Zugabe eines Beta-Galaktosidasestandards in HBSS vollständig hydrolisiert. Anschließend wird ebenfalls das Spektrum der Reaktionsprodukte Chlorphenolrot und o-Nitrophenol im Bereich des sichtbaren Lichtes bestimmt (vergleiche Abbildung 1).

### Beta-Galaktosidase-Assay in wäßrigen Medien

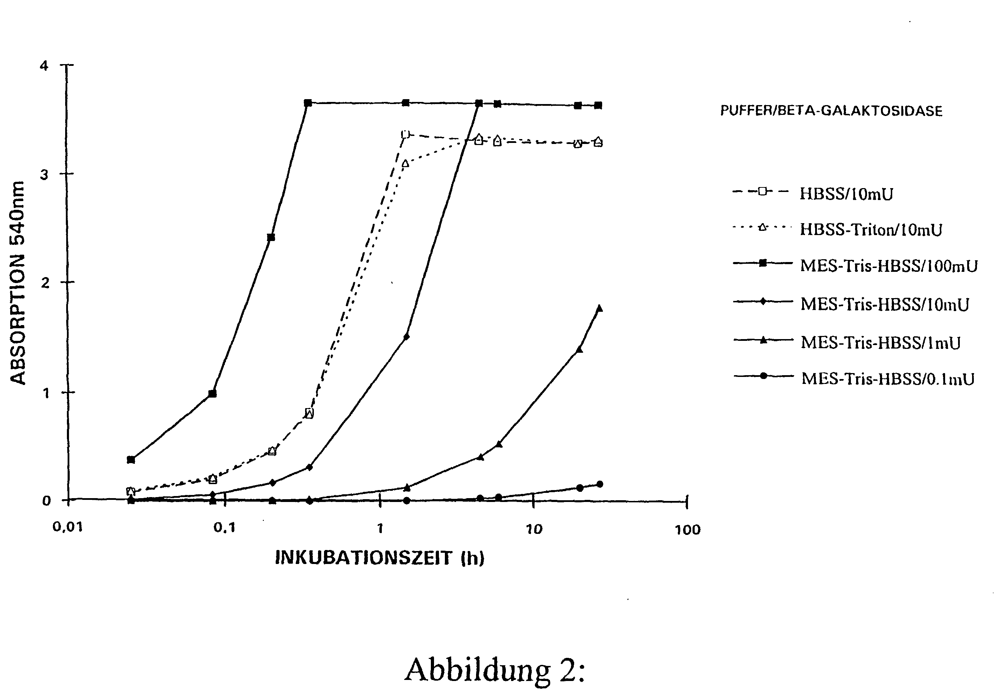

In HBSS werden Verdünnungen eines Beta-Galaktosidasestandards hergestellt und 10µl des Standards werden in einzelne wells einer 96-Well-Platte gegeben. Die Aktivität des Beta-Galaktosidaseenzyms, welches getestet wird, liegt zwischen 100 und 0.0001 mU/Well.
Folgende Puffer kommen zur Anwendung:
HBSS, HBSS mit 0.1 % Triton X-100 (HBSS-TX) sowie 0.02 M MES-Puffer, pH 5.5 mit 0.1 % Triton X-100 (MES-TX).
Zu jedem well mit Beta-Galaktosidasestandard werden 100 µl HBSS oder HBSS-TX bzw. 80µl MES-TX gegeben. Anschließend erfolgt eine Inkubation für 30 min bei 37 °C und 5 % CO₂. Dann werden zu den Wells mit Beta-Galaktosidasestandard und MES-TX Puffer 20µl 0.5 M Tris-HCl gegeben oder 20 µl 0.5, M HEPES (pH 8.0). Darauf erfolgt die Zugabe von 150 µl CPRG Substratlösung mit 1 mg/ml CPRG gelöst in HBSS. Die optische Dichte bei 540-580 nm wird zu verschiedenen Zeitpunkten an einem Mikrotiterplattenphotometer bestimmt (vergleiche Abbildung 2).

### Herstellung der Liposomen:

Lipide werden in Trichlormethan gelöst und danach wird das Trichlormethan durch Evaporation und mehrstündige Vakuumtrocknung entfernt. Die Liposomen werden anschließend durch Zugabe deionisierten Wassers und starkes zweiminütiges Schütteln auf einem Vortexer hergestellt.

### Zellkultur:

MCF-7, MaTu, CC531, 293 und LS174 Zellen werden in RPMI 1640 Medium, F98 Zellen, Bovine Cornea Endothel Zellen in Dulbecco's modifiziertem Eagle's Medium (DMEM), N64, N39, U343 und U373 Zellen in modifiziertem Eagle's Medium (MEM) kultiviert. Alle Medien enthalten 10 % fötales Kälberserum, 100 U/ml Penizillin G, 100 µg/ml Streptomyzinsulfat und 0,25 µg/ml Amphoterizin. Die Zellen werden kultiviert im Zellinkubator mit 5 % CO₂/95 % Luft bei 37 °C.

### Zellzahlbestimmung:

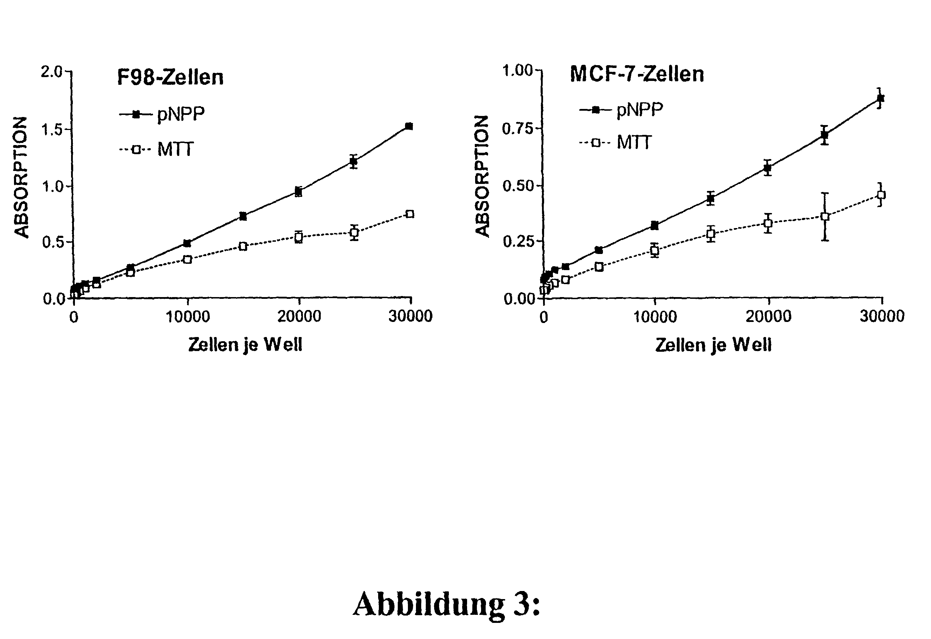

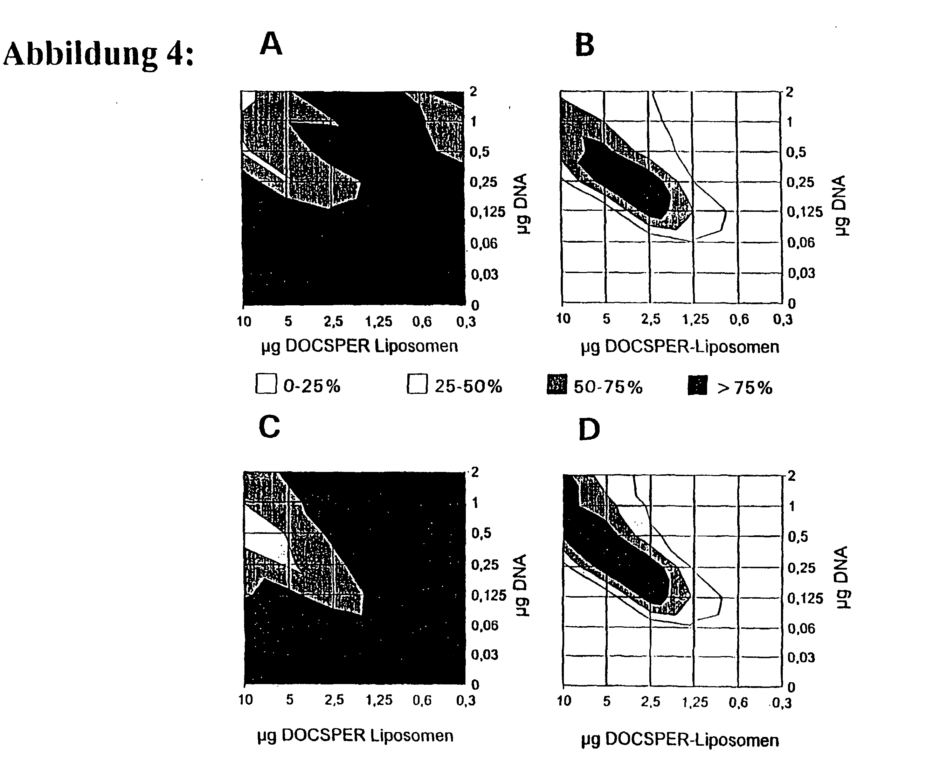

Die Zellen werden trypsiniert, die Zellzahl mit Hilfe der Trypanblaufärbung und der Auszählung mit dem Mikroskop bestimmt. Anschließend werden zwischen 125 und 30.000 Zellen je Well einer 96er Mikrotiterplatte ausgesät. Am folgenden Tag wird das Medium abgenommen, die anhaftenden Zellen einmalig mit Phosphat gepufferter Saline (PBS) gewaschen. Für die Bestimmung der Zellzahl mit Hilfe des MTT-Testes wird 100 µl Medium mit 0.5 mg/ml MTT-Reagenz zugegeben. Nach 3-4 Stunden Inkubation der Zellen bei 37 °C und 5 % CO₂ wird das Medium entfernt und die Formazankristalle werden in Dimethylsulfoxid gelöst. Anschließend erfolgt die Bestimmung der optischen Dichte an einem Mikrotiterplattenphotometer bei 540 nm (Referenzfilter 690nm) (vergleiche Abbildung 3).
Bei Durchführung des saure Phosphatasetestes erfolgt nach dem Waschen der Zellen mit PBS die Zugabe des Substratpuffers 10 mM pNPP in 0.02 M MES/ 0.1 % Triton X-100 (die genauen Volumina für die verschiedenen untersuchten Platten siehe Tabelle 1). Nach 30 min Inkubation bei 37 °C und 5 % CO₂ erfolgt das Abstoppen der Reaktion durch Zugabe von 0.5 M Tris-HCl oder 0.5 M HEPES (pH 8.0). Anschließend erfolgt die Bestimmung der p-Nitrophenolkonzentration durch Bestimmung des Absorption bei 405 nm (Referenzwellenlänge 690 nm) am Mikroplattenmeßgerät.

**Tabelle 1**

| Übersicht über Volumina der verschiedenen Testpuffer auf verschiedenen Zellkulturplatten. | | | |
|---|---|---|---|
| | Saure Phosphatasetestpuffer (µl) | Stoppuffer (µl) | Beta-Galaktosidase testpuffer *(µl) |
| 96-Well-Schale | 80 | 20 | 150 |
| 24-Well-Schale | 240 | 60 | 450 |
| 6-Well-Schale | 800 | 200 | 1500 |

| | | | |
|---|---|---|---|
| *Es können ebenfalls nur 50µl Beta-Galaktosidasetestpuffer für die Bestimmung der Beta-Galaktosidaseaktivität verwendet werden. | | | |

### Bespiele:

- Saure Phosphatasetestpuffer:: 10 mM pNPP in 0.02M MES/0.1% Triton X-100
10 mM pNPP in 0.02M Natriumacetat/0.1% Triton X-100 1mM Naphtylphosphat in 0.02M Natriumacetat/0.1% Triton X-100
- Stoppuffer:: 0.5 M Tris-HCl (pH 8.0)
0.5 M HEPES (pH 8.0)
- Beta-Galaktosidasetestpuffer:: 1 mg/ml CPRG in HBSS (50µl)
2 mg/ml ONPG in HBSS (100µl)
1mM MUG in HBSS (100µl), aus
MUG-Stammlösung 20 mM in DMSO und HBSS

### Transfektion der Zellen:

Für die Gentransfer-Untersuchungen werden 96-Well-Flachbodenzellkulturplatten genutzt. 10.000 bis 20.000 Zellen in 100 µl Medium, mit 10% fötalem Kälberserum werden je Well ausgesät. Am folgenden Tag werden die Lipid/DNA Komplexe wie folgt präpariert (nach Felgner und Mitarbeiter, 1994): Das Lipid wird in einer separaten Platte in serumfreiem Medium zweifach verdünnt von 10µl Lipid per Well beginnend bis zu 0.31 µg Lipid je Well in einem Gesamtvolumen von 50µl. Anschließend wird die DNA separat auf einer anderen 96-Well-Platte ebenfalls zweifach verdünnt, von 2 bis zu 0.03 µg in 50 µl ebenfalls serumfreiem Medium und danach wird die DNA-Verdünnung auf die Zellen pipettiert. Zur Komplexformierung wird die Mischung beider Verdünnungen für etwa 15 bis 30 min bei Raumtemperatur inkubiert. Es erfolgt die Zugabe der Komplexe zu den Zellen. Nach einer Inkubation von 3 bis 5 h werden 100 µl Medium mit 20% fötalem Kälberserum zugegeben und die Zellen werden für 24 bis 72 h zur Expression des applizierten Gens inkubiert.

### Dualer Test von Toxizität und Reportergenexpression

24 bis 72 h nach Transfektion eines lacz-Reportergenkonstruktes werden Zellvitalität bzw. Toxizität der Methode mittels saurem Phosphatasetest bestimmt. Dazu wird das Zellkulturmedium von den Zellen entfernt und die Zellen werden einmalig mit 100 µl PBS gewaschen. Die Bestimmung der Zellvitalität und der Genexpression erfolgt auf verschiedene Weise:

### Beispiel 1:

Nach dem Waschen der Zellen werden 80 µl des Saure Phosphatasetestpuffers hinzugegeben (10 mM pNPP in 0.02 M MES oder 0.02 M Natriumacetat/ 0.1 % Triton X-100 für die Zellyse, pH 5.5). Nach 30 min wird die saure Phosphatasereaktion durch Zugabe von 20µl 0.5 M Tris-HCl (oder 0.5 M HEPES, pH 8.0) abgestoppt. Die Zellzahl wird kalkuliert an Hand der p-Nitrophenolabsorption bei 405 bis 450 nm. Nach der Messung der p-Nitrophenolkonzentration wird der CPRG-Beta-Galaktosidasetestpuffer (Bsp. 50 oder 150 µl 1 mg/ml CPRG in HBSS) zugegeben. Anschließend wird die Expression des lacZ Reportergens, 2 min bis 24 h nach Zugabe des Beta-Galaktosidasetestpuffers, je nach Höhe der Genexpression mit Hilfe der Messung der optischen Absorption bei 540-580 nm des Reaktionsproduktes Chlorphenolrot analysiert. Die Gesamthöhe der Expression kann auf Werte einer Beta-Galaktosidaseeichkurve bezogen werden, die in der gleichen Weise wie die Zellen behandelt wird.

### Beispiel 2 :

Nach dem Waschen der Zellen werden 80 µl des sauren Phosphatasetestpuffers hinzugegeben (10 mM pNPP in 0.02 M MES oder 0.02 M Natriumacetat/ 0.1 % Triton X-100 für die Zellyse, pH 5.5). Nach 30 min wird die saure Phosphatasereaktion durch Zugabe von 20 µl 0.5 M Tris-HCl (oder 0.5 M HEPES, pH 8.0) abgestoppt. Die Zellzahl wird kalkuliert an Hand der p-Nitrophenolabsorption bei 405 bis 450 nm. Nach der Messung der p-Nitrophenolkonzentration wird der ONPG-Beta-Galaktosidasetestpuffer (Bsp. 100 µl 1 mg/ml ONPG in HBSS) zugegeben. Anschließend erfolgt die Analyse der Expression des lacZ Reportergens, 2 min bis 24 h nach Zugabe des ONPG-Beta-Galaktosidasetestpuffers, je nach Höhe der Genexpression, mit Hilfe der Messung der optischen Absorption bei 470 bis 490 nm des Reaktionsproduktes o-Nitrophenol. Die Gesamthöhe der Expression kann auf Werte einer Beta-Galaktosidaseeichkurve bezogen werden, die in der gleichen Weise wie die Zellen behandelt wird.

### Beispiel 3:

Nach dem Waschen der Zellen werden 80 µl des sauren Phosphatasetestpuffers hinzugegeben (10 mM pNPP in 0.02 M MES oder 0.02 M Natriumacetat/0.1 % Triton X-100 für die Zellyse, pH 5.5). Nach 30 min wird die saure Phosphatasereaktion durch Zugabe von 20 µl 0.5 M Tris-HCl (oder 0.5 M HEPES, pH 8.0) abgestoppt. Die Zellzahl wird kalkuliert an Hand der p-Nitrophenolabsorption bei 405 bis 450 nm. Nach der Messung der p-Nitrophenolkonzentration wird der MUG-Beta-Galaktosidasetestpuffers (Bsp. 100 µl 1 mM MUG in HBSS, 5 % DMSO; aus MUG-Stammlösung 20 mM in DMSO und HBSS) zugegeben. Anschließend erfolgt die Analyse der Expression des lacZ Reportergens, 2 min bis 24 h nach Zugabe des MUG-Beta-Galaktosidasetestpuffers, je nach Höhe der Genexpression, mit Hilfe der Messung der Lichtemission des Reaktionsproduktes Methylumbelliferon bei 460-540 nm nach Anregung bei etwa 350 nm. Die Gesamthöhe der Expression kann auf Werte einer Beta-Galaktosidaseeichkurve bezogen werden, die in der gleichen Weise wie die Zellen behandelt wird.

### Beispiel 4:

Nach dem Waschen der Zellen werden 80 µl des sauren Phosphatasetestpuffers hinzugegeben (1 mM Naphtylphosphat in 0.02 M Natriumacetatpuffer/ 0.1 % Triton X-100 für die Zellyse, pH 5.5). Nach 2-4 h wird die saure Phosphatasereaktion durch Zugabe von 20 µl 0.5 M Tris-HCl (oder 0.5 M HEPES, pH 8.0) abgestoppt. Die Zellzahl wird kalkuliert an Hand der Messung der Lichtemission des Reaktionsproduktes Naphtol bei etwa 405 nm nach Anregung bei etwa 350 nm. Nach der Messung der Naphtolkonzentration erfolgt die Zugabe des MUG-Beta-Galaktosidasetestpuffers (Bsp. 100 µl 1 mM MUG in HBSS, 5 % DMSO; aus MUG-Stammlösung 20 mM in DMSO und HBSS). Anschließend erfolgt die Analyse der Expression des lacZ Reportergens, 2 min bis 24 h nach Zugabe des MUG-Beta-Galaktosidasetestpuffers, je nach Höhe der Genexpression, mit Hilfe der Messung der Lichtemission des Reaktionsproduktes Methylumbelliferon bei 460-540 nm nach Anregung bei etwa 350 nm. Die Gesamthöhe der Expression kann auf Werte einer Beta-Galaktosidaseeichkurve bezogen werden, die in der gleichen Weise wie die Zellen behandelt wird.

### Beispiel 5:

Nach dem Waschen der Zellen werden 80 µl des sauren Phosphatasetestpuffers hinzugegeben (10 mM pNPP in 0.02 M Natriumacetatpuffer/ 0.1% Triton X-100 für die Zellyse, pH 5.5). Nach 30 min wird die saure Phosphatasereaktion durch Zugabe von 20µl 0.5 M Tris-HCl (oder 0.5 M HEPES, pH 8.0) abgestoppt. Die Zellzahl wird kalkuliert an Hand der p-Nitrophenolabsorption bei 405 bis 450 nm. Nach der Messung der p-Nitrophenolkonzentration erfolgt die Zugabe des ONPG-MUG-Beta-Galaktosidasetestpuffers. (Bsp. 100µl 1 mM MUG, 7mM ONPG, 5 % DMSO in HBSS; aus MUG-Stammlösung 20 mM in DMSO und HBSS). Anschließend erfolgt die Analyse der Expression des lacZ Reportergens, 2 min bis 24 h nach Zugabe des ONPG-MUG-Beta-Galaktosidasetestpuffers, je nach Höhe der Genexpression.

Die Bestimmung der Genexpression erfolgt dabei
1) mit Hilfe der Messung der Lichtemission des Reaktionsproduktes Methylumbelliferon bei 460-540 nm nach Anregung bei etwa 350 nm und
2) mit Hilfe der Messung der optischen Absorption bei 470 bis 490 nm des Reaktionsproduktes o-Nitrophenol

Die Kombination zweier Substrate ermöglicht das Messen sowohl sehr hoher Beta-Galaktosidase-Konzentrationen mit Hilfe des ONPG-Subbstrates als auch die Bestimmung sehr niedriger Beta-Galactosidasekonzentrationen mit Hilfe des MUG-Substrates. Dadurch wird die Linearität des Testes über einen größeren Bereich ausgedehnt. Die Gesamthöhe der Expression kann auf Werte einer Beta-Galaktosidaseeichkurve bezogen werden, die in der gleichen Weise wie die Zellen behandelt wird.

**Tabelle 2**

| Vergleich der verschiedenen Verfahren zur dualen Bestimmung von Zellvitalität bzw. Toxizität der Gentransfermethode und Effizienz der Gentransfermethode | | | | | |
|---|---|---|---|---|---|
| Beispiel | 1 | 2 | 3 | 4 | 5 |
| Saure Phosphatase-Substrat | pNPP | pNPP | pNPP | Naphtylphosphat | pNPP |
| Saure Phosphatase-Bestimmung | optisch | optisch | optisch | fluorimetrisch | optisch |
| Beta-Galaktosidase-Substrat | CPRG | ONPG | MUG | MUG | MUG/ONPG |
| Beta-Galaktosidase-Bestimmung | optisch | optisch | fluorimetrisch | fluorimetrisch | optisch/ fluorimetrisch |
| Beta-Galaktosidase-Sensitivität | ++ | + | +++ | +++ | +++ |

### Abbildungen

### Abbildung 1:

### Vergleich der Absorptionspektren der Reaktionsprodukte der verwendeten Enzymsubstrate

### Abbildung 2:

Zeitlicher Verlauf der Beta-Galaktosidasereaktion in verschiedenen Puffern. Die Aktivität des Beta-Galaktosidaseenzyms, welches getestet wurde, liegt zwischen 100 und 0,01 mU/Well. Folgende Puffer kamen zur Anwendung: HBSS, HBSS mit 0,1 % Triton X-100 (HBSS-Triton) sowie 0,02 M MES-Puffer, pH 5,5 mit 0,1 % Triton X-100 MES). Zu jedem Well mit Beta-Galaktosidasestandard werden 100 µl HBSS oder HBSS-Triton bzw. 80 µl MES-Puffer gegeben. Anschließend erfolgte eine Inkubation für 30 min bei 37 °C und 5 % CO₂. Dann wurden zu den Wells mit Beta-Galaktosidasestandard und MES-Puffer 20 µl 0,5 M Tris-HCl (pH 8,0) gegeben. Darauf erfolgte die Zugabe von 150 µl CPRG Substratlösung mit 1 mg/ml CPRG gelöst in HBSS. Die optische Dichte wurde bei 540-580 nm zu verschiedenen Zeitpunkten an einem Mikrotiterplattenphotometer bestimmt.

### Abbildung 3:

Vergleich von MTT-Assay nach Mosmann und dem Saure Phosphatase-Assay bei der Zellzahlbestimmung. Durchführung des Saure Phosphatase-Assay wie im Text angegeben. Puffer: 80 µl 10 mM pNPP in 0,02 M MES mit 0,1 % Triton X-100 (pH 5,5), 20 µl Tris-HCl (pH 8,0) bei der Zellzahlbestimmung.

### Abbildung 4:

Vergleich von MTT-Assay nach Mosmann (A) und dem Beta-Galaktosidase-Assay nach Lim und Chae (B) mit dem dualen Assay (C+D;C: Ergebnisse des Saure Phosphatase-Assay; D: Beta-Galaktosidase-Assay nach Saure Phosphatase-Assay).
MCF-7 Zellen wurden mit DOCSPER-Liposomen und der Plasmid-DNA pUT 651 (Cayla, Toulouse, Frankreich) wie bei Felgner und Mitarbeitern (1994) beschrieben transfiziert. 3 Tage nach der Transfektion erfolgte die Bestimmung von Zellvitalität und Beta-Galaktosidaseexpression wie in Beispiel 1 (80 µl 10 mM pNPP in 0,02 M MES; 0,1 % Triton X-100 (pH 5,5), 20 µl Tris-HCl (pH 8,0), 150 µl 1 mg/ml CPRG in HBSS) angegeben. Die Zellvitalität von Kontrollzellen und die maximale Beta-Galaktosidaseexpression des Experiments wurden auf 100 % gesetzt.

## Patentansprüche

1. Verfahren zur parallelen Bestimmung von Zellvitalität und Effizienz nach Gentransfer in eukaryontische Zellen,
**dadurch gekennzeichnet, daß** man
die Zellzahl der transfizierten Zellen durch eine repräsentative Enzymaktivität mittels Bestimmung der Sauren Phosphatase ermittelt und nachfolgend im selben Reaktionsgefäß die Effizienz des Gentransfers durch colorimetrische oder fluorimetrische Bestimmung der Reportergenaktivität nach Änderung der Ionenkonzentration und des pH-Wertes.

2. Verfahren nach Anspruch 1,
**dadurch gekennzeichnet, daß** man
zur Effizienzbestimmung die Pufferstärken erniedrigt.

3. Duales Testsystem zur Bestimmung von Zellvitalität und Effizienz nach Gentransfer in eukaryontische Zellen umfassend
a) einen Vitalitäts-Assay mit
einem Substrat zur Bestimmung Saurer Phosphatase und einem Puffer für die Zellyse, wobei als Substrat pNPP oder Naphthylphosphat verwendet wird und der verwendete Puffer eine niedrige Ionenstärke besitzt, die nach Bestimmung der Saure Phosphataseaktivität eine Veränderung des pH-Wertes vom sauren in den basischen pH-Bereich ermöglicht, und
b) einem Substrat zur colorimetrischen oder fluorimetrischen Bestimmung der β-Galaktosidase-Aktivität als Reportengen-Assay zur Effizienzbestimmung.

## Claims

1. Method for parallel determination of cell vitality and efficiency following gene transfer into eucaryontic cells,
wherein
the cell count of the transfected cells is determined by a representative enzyme activity by means of determination of the acid phosphatase followed in the same reaction vessel by the efficiency of the gene transfer by colorimetric or fluorimetric determination of the reporter gene activity after alteration of the ion concentration and the pH figure.

2. Method according to Claim 1,
wherein
the buffer strengths are reduced to determine the efficiency.

3. Dual test system to determine cell vitality and efficiency following gene transfer into eucaryontic cells
entailing
a) a vitality assay with a substrate for the determination of acid phosphatase and a buffer for the cell lysis, with pNPP or naphthyl phosphate being used as the substrate and the buffer used having a low ion strength, enabling an alteration of the pH figure from the acid to the basic pH area after determination of the acid phosphatase activity, and
b) a substrate for the colorimetric or fluorimetric determination of the β-galactosidase activity as a reporter gene assay for determination of the efficiency.

## Revendications

1. Procédé destiné à déterminer parallèlement la vitalité cellulaire et l'efficacité après un transfert génétique dans des cellules eucaryotiques,
**se caractérisant par le fait que**
l'on détermine le nombre de cellules transférées par le biais d'une activité enzymatique représentative au moyen de la détermination de la phosphatase acide et ensuite dans le même réacteur l'efficacité du transfert génétique par le biais de la détermination colorimétrique ou fluorimétrique de l'activité du gène reporter après modification de la concentration en ions et de la valeur du pH.

2. Procédé selon la revendication 1,
**se caractérisant par le fait que**
l'on réduit les forces tampons pour déterminer l'efficacité.

3. Système de test dual destiné à déterminer la vitalité cellulaire et l'efficacité après un transfert génétique dans des cellules eucaryotiques
comprenant
a) un test de vitalité avec
un substrat permettant de déterminer la phosphatase acide et un tampon pour la lyse cellulaire, le substrat utilisé étant le pNPP ou le naphtylphosphate et le tampon employé possédant une force ionique peu élevée, ce qui permet après détermination de l'activité de la phosphatase acide, de modifier la valeur du pH de la zone acide dans la zone du pH basique et
b) un substrat permettant la détermination colorimétrique ou fluorimétrique de l'activité de la ß-galactosidase en tant que test du gène reporter pour déterminer l'efficacité.
